# EUROPEAN PATENT SPECIFICATION

(11) **EP 1 877 552 B1**
(45) Date of publication and mention of the grant of the patent: **14.07.2010**
(21) Application number: 06796825.5
(22) Date of filing: 21.08.2006
(51) Int. Cl.: C12N 9/88, C12N 15/09, C12N 15/81, C12C 11/00, C12C 11/02

(54) **O-ACETYLHOMOSERINESULFHYDRYLASE GENE AND USE THEREOF**
O-ACETYLHOMOSERINSULFHYDRYLASE GEN UND VERWENDUNGEN DAVON
GENE DE O-ACETYLHOMOSERINESULFHYDRYLASE ET UTILISATION DE CELUI-CI

(30) Priority: 22.08.2005 JP 2005240351; 23.02.2006 JP 2006047564
(43) Date of publication of application: 16.01.2008
(73) Proprietor: Suntory Holdings Limited, Kita-ku, Osaka-shi, Osaka 530-8203 (JP)
(72) Inventor: NAKAO, Yoshihiro, Mishima-gun, Osaka 618-8503 (JP); KODAMA, Yukiko, Mishima-gun, Osaka 618-8503 (JP); SHIMONAGA, Tomoko c/o Suntory Limited, Mishima-gun Osaka 6188503 (JP)
(74) Representative: Vossius & Partner
(86) International application number: PCT/JP2006/316781
(87) International publication number: WO 2007/023969

(56) References cited:
- EP-A2- 0 699 748
- KERJAN P ET AL: "NUCLEOTIDE SEQUENCE OF THE SACCHAROMYCES CEREVISIAE MET 25 GENE" NUCLEIC ACIDS RESEARCH, OXFORD UNIVERSITY PRESS, SURREY, GB, vol. 14, no. 20, 1986, pages 7861-7871, XP002051281 ISSN: 0305-1048 & DATABASE EMBL [Online] 7 June 1987 (1987-06-07), "Yeast MET25 gene for OAH-OAS sulfhydrylase (= O-acetyl homoserine - O-acetyl serine sulfhydrylase)" retrieved from EBI accession no. EM_PRO:X04493 Database accession no. X04493
- OMURA F ET AL: "REDUCTION OF HYDROGEN SULFIDE PRODUCTION IN BREWING YEAST BY CONSTITUTIVE EXPRESSION OF MET25 GENE" JOURNAL OF THE AMERICAN SOCIETY OF BREWING CHEMISTS, AMERICAN SOCIETY OF BREWING CHEMISTS, ST PAUL, MN, US, vol. 53, no. 2, 1995, pages 58-62, XP002066284 ISSN: 0361-0470
- TEZUKA H ET AL: "CLONING OF A GENE SUPPRESSING HYDROGEN SULFIDE PRODUCTION BY SACCHAROMYCES CEREVISIAE AND ITS EXPRESSION IN A BREWING YEAST" JOURNAL OF THE AMERICAN SOCIETY OF BREWING CHEMISTS, AMERICAN SOCIETY OF BREWING CHEMISTS, ST PAUL, MN, US, vol. 50, 1992, pages 130-133, XP002066285 ISSN: 0361-0470

## Description

### TECHNICAL FIELD

The present invention relates to an O-acetylhomoserinesulfhydrylase gene and to uses of the gene. The invention relates in particular to a brewer's yeast which produces alcoholic beverages of excellent flavor, alcoholic beverages produced using such a yeast, and a method of producing such alcoholic beverages. More specifically, the invention relates to MET17 gene which codes for the O-acetylhomoserinesulfhydrylase Met17p in brewer's yeast, particularly to a yeast which improves the flavor of product by increasing the level of expression of the non-ScMET17 gene characteristic to beer yeast and to a method of producing alcoholic beverages using such a yeast.

### BACKGROUND ART

The beer yeast used in the production of commercial Pilsner-type light-colored beers has the property of forming hydrogen sulfide during the primary fermentation step. This hydrogen sulfide is one cause of the immature beer aroma that is undesirable to beer quality. To reduce this aroma below a threshold level, extension of the secondary fermentation and maturation periods is carried out.

Research on the factors affecting the formation of hydrogen sulfide, conducted with the aim of lowering the level of hydrogen sulfide in beer (Jangaard, N.O., Gress, H.S. and Coe, RW.: Amer. Soc. Brew. Chem. Proc., p. 46 (1973); Kuroiwa, Y. and Hashimoto, N.: Brew. Dig., 45, 44 (1970); Hysert, D.W. and Morrison, N.M.: J. Amer. Soc. Brew. Chem, 34, 25 (1976)), and research on the development of a low-hydrogen sulfide-producing yeast using a mutation process or a cell fusion process (Molzahm, S.W.: J. Amer. Soc. Brew. Chem., 35, 54 (1977)) have been reported in the literature.

In addition to reducing the amount of hydrogen sulfide produced by yeast, each of these approaches also affects the other brewing properties of the yeast (fermentation rate, beer flavor). Hence, such a yeast well-suited for brewing beer has yet to be achieved. Recently, the development of brewer's yeasts using genetic engineering technology has been carried out Japanese Patent Application Laid-open No. H5-244955 discloses that a beer yeast in which a DNA fragment coding for cystathionine β-synthase has been inserted reduces the production of hydrogen sulfide. However, the degree of reduction is small, with the amount of hydrogen sulfide produced by the transformant being about 60 to 80% of that by the parent strain.

In yeast metabolism, hydrogen sulfide is produced in the process of reducing sulfate ions (SO₄²-) taken up from the medium. This metabolic system is a pathway fur the biosynthesis of sulfur-containing amino acids such as methionine and cysteine. Detailed studies have been published on the enzyme that takes part at each stage of the pathway, and on its gene (the MET17 gene) (see Tabor, H. and Tabor, C.W., eds., Methods in Enzymology, Vol 17B (London: Academic Press, 1971); and Jakoby, W.B. and Griffith, O.W., eds., Methods in Enzymology, Vol. 143 (London: Academic Press, 1987)).

O-acetylhomoserinesulfhydrylase is an enzyme which transfers a sulfur atom from hydrogen sulfide to O-acetylhomoserine, and is encoded by the MET17 gene. This enzymes also transfers a sulfur atom to O-acetylserine. It has been reported that, with a beer yeast strain in which the MET17 gene from *Saccharomyces cerevisiae* X2180-1A has been constitutively expressed, the amount of hydrogen sulfide produced fulls to about 2% of the level in the parent strain (Japanese Patent Application Laid-open No. H7-303475).

### DISCLOSURE OF INVENTION

The invention relates to the embodiments as characterised in the claims.

As noted above, variant strains have been developed in order to lower the amount of hydrogen sulfide produced in the final product. As a result, unexpected delays in fermentation and increases in undesirable flavor components have been observed in some cases, making the practical use of such yeasts questionable. A desire has thus existed for a method of developing yeasts which produce less hydrogen sulfide without compromising either the fermentation rate or the product quality.

The materials and methods disclosed herein solve the above problems, and as a result succeeded in identifying and isolating a gene encoding O-acetrylhomoserinesulfhydrylase from lager brewing yeast. Moreover, a yeast was transformed by introducing and expressing with the obtained gene to confirm that the amount of hydrogen sulfide produced was reduced, thereby completing the present invention.

Thus, the present invention relates to a novel O-acetylhomoserinesulfhydrylase gene existing specifically in a lager brewing yeast, to a protein encoded by said gene, to a transformed yeast in which the expression of said gene is controlled, to a method for controlling the amount of hydrogen sulfide in a product by using a yeast in which the expression of said gene is controlled. More specifically, the present invention provides the following polynucleotides, a vector comprising said polynucleotide, a transformed yeast introduced with said vector, a method for producing alcoholic beverages by using said transformed yeast, and the like.
(1) A polynucleotide selected from the group consisting of
   (a) a polynucleotide comprising the nucleotide sequence of SEQ ID NO:1;
(2) The polynucleotide of (1) above comprising a polynucleotide encoding a protein consisting of SEQ ID NO: 2.
(3) The polynucleotide of any one of (1) to (4) above, wherein the polynucleotide is DNA.
(4) A protein encoded by the polynucleotide of any one of (1) to (3) above.
(5) A vector comprising the polynucleotide of any one of (1) to (3) above.
(5a) The vector of (5) above, which comprises the expression cassette comprising the following components:
   (x) a promoter that can be transcribed in a yeast cell;
   (y) any of the polynucleotides described in (1) to (3) above linked to the promoter in a sense or antisense direction; and
   (z) a signal that can function in a yeast with respect to transcription termination and polyadenylation of a RNA molecule.
(6) A yeast, wherein the vector of (5) above is introduced.
(7) The yeast of (6) above, wherein hydrogen sulfide-producing ability is reduced by introducing the vector of (5) above.
(8) The yeast of (7) above, wherein a hydrogen sulfide-producing ability is reduced by increasing an expression level of the protein of (4) above.
(9) A method for producing an alcoholic beverage by using the yeast of any one of (6) through (8) above.
(10) The method for producing an alcoholic beverage of (9) above, wherein the brewed alcoholic beverage is a malt beverage.
(11) The method for producing an alcoholic beverage of (9) above, wherein the brewed alcoholic beverage is wine.
(12) A method for assessing a test yeast for its hydrogen sulfide-producing ability, comprising designing a primer or a probe based on the nucleotide sequence of SEQ ID NO: 1 and using the primer or probe designed to assess the test yeast for its hydrogen sulfide-producing capability.
(12a) A method for selecting a yeast having a low hydrogen sulfide-producing ability by using the method in (12) above.
(12b) A method for producing an alcoholic beverage (for example, beer) by using the yeast selected with the method in (12a) above.
(13) A method for assessing a test yeast for its hydrogen sulfide-producing capability, comprising: culturing a test yeast; and measuring an expression level of an O-acetylhomoserinesulfhydrylase gene having the nucleotide sequence of SEQ ID NO: 1.
(13a) A method for selecting a yeast having a low hydrogen sulfide-producing ability, which comprises assessing a test yeast by the method described in (13) above and selecting a yeast having a high expression level of O-acetylhomoserinesulfhydrylase gene.
(13b) A method for producing an alcoholic beverage (for example, beer) by using the yeast selected with the method in (13a) above.
(14) A method for selecting a yeast, comprising: culturing test yeasts; quantifying the protein of (4) above or measuring an expression level of an O-acetylhomoserinesulfhydrylase gene having the nucleotide sequence of SEQ ID NO: 1; and selecting a test yeast having said protein amount or said gene expression level according to a target capability of producing hydrogen sulfide.
(14a) A method for selecting a yeast, comprising: culturing test yeasts; measuring hydrogen sulfide-producing ability or O-acetylhomoserinesulfhydrylase activity; and selecting a test yeast having a target capability of producing hydrogen sulfide or a target O-acetylhomoserinesulfhydrylase activity.
(15) The method for selecting a yeast of (14) above, comprising: culturing a reference yeast and test yeasts; measuring an expression level of an O-acetylhomoserinesulflydrylase gene having the nucleotide sequence of SEQ ID NO: 1 in each yeast; and selecting a test yeast having the gene expressed higher than that in the reference yeast.
(16) The method for selecting a yeast of (14) above comprising: culturing a reference yeast an test yeasts; quantifying the protein of (4) above in each yeast; and selecting a test yeast having said protein for a larger amount than that in the reference yeast. That is, the method for selecting a yeast of (14) above comprising: culturing plural yeasts; quantifying the protein of (4) above in each yeast; and selecting a test yeast having a large amount of the protein from them.
(17) A method for producing an alcoholic beverage comprising: conducting fermentation for producing an alcoholic beverage using the yeast according to any one of (6) to (8); and adjusting the production amount of hydrogen sulfide.

According to the method for producing alcoholic beverages by using a yeast transformed with an O-acetylhomoserinesulfhydrylase, hydrogen sulfide is consumed quickly and then the concentration of hydrogen sulfide can be lowered in beer fermentation and the finished product so that alcoholic beverages can be produced with enhanced flavor.

### BRIEF DESCRIPTION OF DRAWINGS

Figure 1 shows the cell growth with time upon beer fermentation test. The horizontal axis represents fermentation time while the vertical axis represents optical density at 660 nm (OD660).
Figure 2 shows the sugar consumption with time upon beer fermentation test. The horizontal axis represents fermentation time while the vertical axis represents apparent extract concentration (w/w%).
Figure 3 shows the expression profile of non-ScMET17 gene in yeasts upon beer fermentation test. The horizontal axis represents fermentation time while the vertical axis represents the intensity of detected signal.
Figure 4 shows the cell growth with time upon Fermentation test using parent strain and non-ScMET17 highly expressed strain. The horizontal axis represents fermentation time while the vertical axis represents optical density at 660 nm (OD660).
Figure 5 shows the sugar consumption with time upon beer fermentation test using parent strain and non-ScMET17 highly expressed stain. The horizontal axis represents fermentation time while the vertical axis represents apparent extract concentration (w/w%).

### BEST MODES FOR CARRYING OUT THE INVENTION

The present inventors conceived that it is possible to lower hydrogen sulfide effectively by increasing O-acetylhomoserinesulfhydrylase activity of the yeast. The present inventors have studied based on this conception and as a result, isolated and identified non-ScMET17 gene encoding an O-acetylhomoserinesulfhydrylase unique to lager brewing yeast based on the lager brewing yeast genome information mapped according to the method disclosed in Japanese Patent Application Laid-Open No. 2004-283169. The nucleotide sequence of the gene is represented by SEQ ID NO: 1. Further, an amino acid sequence of a protein encoded by the gene is represented by SEQ ID NO: 2.

### 1. Polynucleotide of the invention

First of all, the specification describes a polynucleotide comprising the nucleotide sequence of SEQ ID NO:1; and (b) a polynucleotide comprising a polynucleotide encoding a protein of the amino acid sequence of SEQ ID NO:2. The polynucleotide can be DNA or RNA.

The target polynucleotide of the specification is not limited to the polynucleotide encoding an O-acetylhomoserinesulfhydrylase gene derived from lager brewing yeast and may include other polynucleotides encoding proteins having equivalent functions to said protein. Proteins with equivalent functions include, for example, (c) a protein of an amino acid sequence of SEQ ID NO: 2 with one or more amino acids thereof being deleted, substituted, inserted and/or added and having O-acetylhomoserinesulfhydrylase activity.

Such proteins include a protein consisting of an amino acid sequence of SEQ ID NO: 2 with, for example, 1 to 100, 1 to 90, 1 to 80, 1 to 70, 1 to 60, 1 to 50, 1 to 40, 1 to 39, 1 to 38, 1 to 37, 1 to 36, 1 to 35, 1 to 34, 1 to 33, 1 to 32, 1 to 31, 1 to 30, 1 to 29, 1 to 28, 1 to 27, 1 to 26, 1 to 25, 1 to 24, 1 to 23, 1 to 22, 1 to 21, 1 to 20, 1 to 19, 1 to 18, 1 to 17, 1 to 16, 1 to 15, 1 to 14, 1 to 13, 1 to 12, 1 to 11, 1 to 10, 1 to 9, 1 to 8, 1 to 7, 1 to 6 (1 to several amino acids), 1 to 5, 1 to 4, 1 to 3; 1 to 2, or 1 amino acid residues thereof being deleted, substituted, inserted and/or added and having an O-acetylhomoserinesulfhydrylase activity. In general, the number of deletions, substitutions, insertion, and/or additions is preferably smaller. In addition, such proteins include (d) a protein having an amino acid sequence with about 60% or higher, about 70% or higher, 71% or higher, 72% or higher, 73% or higher, 74% or higher, 75% or higher, 76% or higher, 77% or higher, 78% or higher, 79% or higher, 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, . 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher identity with the amino acid sequence of SEQ ID NO: 2, and having an O-acetylhomoserinesulfhydrylase activity. In general, the percentage identity is preferably higher.

O-acetylhomoserinesulfhydrylase activity may be measured, for example, by a method of Thomas et al as described in Yeast. 9(12): 1335-42, 1993.

Furthermore, the specification also contemplates (e) a polynucleotide comprising a polynucleotide which hybridizes to a polynucleotide consisting of a nucleotide sequence complementary to the nucleotide sequence of SEQ ID NO: 1 under stringent conditions and which encodes a protein having O-acetylhomoserinesulfhydrylase activity; and (f) a polynucleotide comprising a polynucleotide which hybridizes to a polynucleotide complementary to a nucleotide sequence of encoding a protein of SEQ ID NO: 2 under stringent conditions, and which encodes a protein having O-acetylhomoserinesullhydrylase activity.

Herein, "a polynucleotide that hybridizes under stringent conditions" refers to nucleotide sequence, such as a DNA, obtained by a colony hybridization technique, a plaque hybridization technique, a southern hybridization technique or the like using all or part of polynucleotide of a nucleotide sequence complementary to the nucleotide sequence of SEQ ID NO: 1 or polynucleotide encoding the amino acid sequence of SEQ ID NO: 2 as a probe. The hybridization method may be a method described, for example, in MOLECULAR CLONING 3rd Ed., CURRENT PROTOCOLS IN MOLECULAR BIOLOGY, John Wiley & Sons 1987-1997.

The term "stringent conditions," as used herein may be any of low stringency conditions, moderate stringency conditions or high stringency conditions. "Low stringency conditions" are, for example, 5 x SSC, 5 x Denhardt's solution, 0.5% SDS, 50% formamide at 32°C. "Moderate stringency conditions" are, for example, 5 x SSC, 5 x Denhardt's solution, 0.5% SDS, 50% formamide at 42°C. "High stringency conditions" are, for example, 5 x SSC, 5 x Denhardt's solution, 0.5% SDS, 50% formamide at 50°C. Under these conditions, a polynucleotide, such as a DNA, with higher homology is expected to be obtained efficiently at higher temperature, although multiple factors are involved in hybridization stringency including temperature, probe concentration, probe length, ionic strength, time, salt concentration and others, and one skilled in the art may appropriately elect these factors to realize similar stringency.

When a commercially available kit is used for hybridization, for example, Alkphos Direct Labeling Reagents (Amersham Pharmacia) may be used. In this case, according to the attached protocol, after incubation with a labeled probe overnight, the membrane is washed with a primary wash buffer containing 0.1% (w/v) SDS at 55°C, thereby detecting hybridized polynucleotide, such as DNA.

Other polynucleotides that can be hybridized include polynucleotides having about 60% or higher, about 70% or higher, 71% or higher, 72% or higher, 73% or higher, 74% or higher, 75% or higher, 76% or higher, 77% or higher, 78% or higher, 79% or higher, 80% or higher, 81% or higher, 82% or higher, 83% or higher, 84% or higher, 85% or higher, 86% or higher, 87% or higher, 88% or higher, 89% or higher, 90% or higher, 91 % or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher or 99.9% or higher identity to polynucleotide encoding the amino acid sequence of SEQ ID NO: 2 as calculated by homology search software, such as FASTA and BLAST using default parameters.

Identity between amino acid sequences or nucleotide sequences may be determined using algorithm BLAST by Karlin and Altschul (Proc. Natl. Acad Sci. USA, 87: 2264-2268, 1990; Pro. Natl. Acad. Sci. USA, 90: 5873,1993). Programs called BLASTN and BLASTX based on BLAST algorithm have been, developed (Altschul SF et aL, J. Mol. Biol. 215: 403, 1990). When a nucleotide sequence is sequenced using BLAST, the parameters are, for example, score =100 and word length = 12. When an amino acid sequence is sequenced using BLASTX, the parameters are, for example, score = 50 and word length = 3. When BLAST and Gapped BLAST programs are used, default parameters for each of the programs are employed.

### 2. Protein of the present invention

The present specification also provides proteins encoded by any of the polynucleotides (a) to (f) above. A preferred protein of the present invention comprises an amino acid sequence of SEQ ID NO:2 with one or several amino acids thereof being deleted, substituted, inserted and/or added, and has O-acetylhomoserinesullhydrylase activity.

Such protein includes those having an amino acid sequence of SEQ ID NO: 2 with amino acid residues thereof of the number mentioned above being deleted, substituted, inserted and/or added and having an O-acetylhomoserinesullhydrylase activity. In addition, such protein includes those having homology as described above with the amino acid sequence of SEQ ID NO: 2 and having O-acetylhomoserinesullhydrylase activity.

Such proteins may be obtained by employing site-directed mutation described, for example, in MOLECULAR CLONING 3rd Ed, CURRENT PROTOCOLS IN MOLECULAR BIOLOGY, Nuc. Acids. Res., 10: 6487 (1982), Proc. Natl. Acad Sci. USA 79: 6409 (1982), Gene 34: 315 (1985), Nuc Acids. Res., 13: 4431 (1985), Proc. Natl. Acad Sci. USA 82: 488 (1985).

Deletion, substitution, insertion and/or addition of one or more amino acid residues in an amino acid sequence of the protein of the specification means that one or more amino acid residues are deleted substituted, inserted and/or added at any one or more positions in the same amino acid sequence. Two or more types of deletion, substitution, insertion and/or addition may occur concurrently.

Hereinafter, examples of mutually substitutable amino acid residues are enumerated Amino acid residues in the same group are mutually substitutable. The groups are provided below.

Group A: leucine, isoleucine, norleucine, valine, norvaline, alanine, 2-aminobutanoic acid, methionine, o-methylserine, t-butylglycine, t-butylalanine, cyclohexylalanine; Group B: asparatic acid, glutamic acid, isoasparatic acid, isoglutamic acid, 2-aminoadipic acid, 2-aminosuberic acid; Group C: asparagine, glutamine; Group D: lysine, arginine, ornithine, 2,4-diaminobutanoic acid, 2,3-diaminopropionic acid; Group E: proline, 3-hydroxyproline, 4-hydroxyproline; Group F: serine, threonine, homoserine; and Group G: phenylalanine, tyrosine.

The protein of the present invention may also be produced by chemical synthesis methods such as Fmoc method (fluorenylmethyloxycarbonyl method) and tBoc method (t-butyloxycarbonyl method). In addition, peptide synthesizers available from, for example, Advanced ChemTech, PerkinElmer, Pharmacia, Protein Technology Instrument, Synthecell-Vega, PerSeptive, Shimazu Corp. can also be used for chemical synthesis.

### 3. Vector of the invention and yeast transformed with the vector

The present invention then provides a vector comprising the polynucleotide of the invention. Generally, the vector of the present invention comprises an expression cassette including as components (x) a promoter that can transcribe in a yeast cell; (y) a polynucleotide described in any of (a) to (i) above that is linked to the promoter in sense or antisense direction; and (z) a signal that functions in the yeast with respect to transcription termination and polyadenylation of RNA molecule. According to the present invention, in order to highly express the protein of the invention described above upon brewing alcoholic beverages (e.g., beer) described below, these polynucleotides are introduced into the promoter in the sense direction to promote expression of the polynucleotide (DNA) described in any of (a) to (i) above.

A vector introduced in the yeast may be any of a multicopy type (YEp type), a single copy type (YCp type), or a chromosome integration type (YIp type). For example, YEp24 (J. R Broach et al., EXPERIMENTAL MANIPULATION OF GENE EXPRESSION, Academic Press, New York, 83, 1983) is known as a YEp type vector, YCp50 (M. D. Rose et al., Gene 60: 237, 1987) is known as a YCp type vector, and YIp5 (K. Struhl et al., Proc. Natl. Acad. Sci. USA, 76: 1035, 1979) is known as a YIp type vector, all of which are readily available.

Promoters/terminators for adjusting gene expression in yeast may be in any combination as long as they function in the brewery yeast and they are not influenced by constituents in fermentation broth. For example, a promoter of glyceraldehydes 3-phosphate dehydrogenase gene (TDH3), or a promoter of 3-phosphoglycerate kinase gene (PGKI) may be used. These genes have previously been cloned, described in detail, for example, in M. F. Tuite et al., EMBO J., 1, 603 (1982), and are readily available by known methods.

Since an auxotrophy marker cannot be used as a selective marker upon transformation for a brewery yeast, for example, a geneticin-resistant gene (G418r), a copper-resistant gene (CUP1) (Marin et al., Proc. Natl. Acad. Sci. USA, 81, 337 1984) or a cerulenin-resistant gene (fas2m, PDR4) (Junji Inokoshi et- al., Biochemishy, 64, 660, 1992; and Hussain et al., Gene, 101: 149, 1991, respectively) may be used.

A vector constructed as described above is introduced into a host yeast. Examples of the host yeast include any yeast that can be used for brewing, for example, brewery yeasts for beer, wine and sake. Specifically, yeasts such as genus *Saccharomyces* may be used. According to the present invention, a lager brewing yeast, for example, *Saccharomyces pastorianus* W34/70, *Saccharomyces carlsbergensis* NCYC453 or NCYC456, or *Saccharomyces cerevisiae* NBRC1951, NBRC1952, NBRC1953 or NBRC1954 may be used. In addition, whisky yeasts such as *Saccharomyces cerevisiae* NCYC90, wine yeasts such as wine yeasts #1, 3 and 4 from the Brewing Society of Japan, and sake yeasts such as sake yeast #7 and 9 from the Brewing Society of Japan may also be used but not limited thereto. In the present invention, lager brewing yeasts such as *Saccharomyces pastorianus* may be used preferably.

A yeast transformation method may be a generally used known method. For example, methods that can be used include but not limited to an electroporation method *(*Meth. Enzym., 194: 182 (1990)), a spheroplast method *(*Proc.Natl. Acad. Sci. USA, 75: 1929(1978)), a lithium acetate method *(*J. Bacteriology, 153:163 (1983)), and methods descnbed *in* Proc. Natl. Acad. Sci. USA, 75: 1929 (1978), METHODS IN YEAST GENETICS, 2000 Edition: A Cold Spring Harbor Laboratory Course Manual.

More specifically, a host yeast is cultured in a standard yeast nutrition medium (e.g., YEPD medium (Genetic Engineering. Vol. 1, Plenum Press, New York, 117(1979)), etc.) such that OD600 nm will be 1 to 6. This culture yeast is collected by centrifugation, washed and pre-treated with alkali ion metal ion, preferably lithium ion at a concentration of about 1 to 2 M. After the cell is left to stand at about 30°C for about 60 minutes, it is left to stand with DNA to be introduced (about 1 to 20 µg) at about 30°C for about another 60 minutes. Polyethyleneglycol, preferably about 4,000 Dalton of polyethyleneglycol is added to a final concentration of about 20% to 50%. After leaving at about 30°Cfur about 30 minutes, the cell is heated at about 42°C for about 5 minutes. Preferably, this cell suspension is washed with a standard yeast nutrition medium, added to a predetermined amount of fresh standard yeast nutrition medium and left to stand at about 30°C for about 60 minutes. Thereafter, it is seeded to a standard agar medium containing an antibiotic or the like as a selective marker to obtain a transformant.

Other general cloning techniques may be found, for example, in MOLECULAR CLONING 3rd Ed., and METHODS IN YEAST GENETICS, A LABORATORY MANUAL (Cold Spring Harbor Laboratory Press, Cold Spring Harbor, NY).

### 4. Method of producing alcoholic beverages according to the present invention and alcoholic beverages produced by the method

The vector of the present invention described above is introduced into a yeast suitable for brewing a target alcoholic product. This yeast can be used to produce a desired alcoholic beverage with enhanced flavor with a lowered content of hydrogen sulfide. In addition, yeasts to be selected by the yeast assessment method of the present invention described below can also be used The target alcoholic beverages include, for examples, but not limited to beer, sparkling liquor *(happoushu)* such as a beer-taste beverage, wine, whisky, sake and the like.

In order to produce these alcoholic beverages, a known technique can be used except that a brewery yeast obtained according to the present invention is used in the place of a parent strain. Since materials, manufacturing equipment, manufacturing control and the like may be exactly the same as the conventional ones, there is no need of increasing the cost for producing alcoholic beverages with a lowered content of hydrogen sulfide. Thus, according to the present invention, alcoholic beverages with enhanced flavor can be produced using the existing facility without increasing the cost.

### 5. Yeast assessment method of the invention

The present invention relates to a method for assessing a test yeast for its hydrogen sulfide-producing capability by using a primer or a probe designed based on a nucleotide sequence of an O-acetylhomoserinesulfhydrylase gene having the nucleotide sequence of SEQ ID NO:1. General techniques for such assessment method is known and is described in, for example, WO01/040514, Japanese Laid-Open Patent Application No. 8-205900 or the like. This assessment method is described in below.

First, genome of a test yeast is prepared. For this preparation, any known method such as Hereford method or potassium acetate method may be used (e.g., METHODS IN YEAST GENETICS, Cold Spring Harbor Laboratory Press, 130 (1990)). Using a primer or a probe designed based on a nucleotide sequence (preferably, ORF sequence) of the O-acetylhomoserinesulfhydrylase gene, the existence of the gene or a sequence specific to the gene is determined in the test yeast genome obtained. The primer or the probe may be designed according to a known technique.

Detection of the gene or the specific sequence may be carried out by employing a known technique. For example, a polynucleotide including part or all of the specific sequence or a polynucleotide including a nucleotide sequence complementary to said nucleotide sequence is used as one primer, while a polynucleotide including part or all of the sequence upstream or downstream from this sequence or a polynucleotide including a nucleotide sequence complementary to said nucleotide sequence, is used as another primer to amplify a nucleic acid of the yeast by a PCR method, thereby determining the existence of amplified products and molecular weight of the amplified products. The number of bases of polynucleotide used for a primer is generally 10 base pairs (bp) or more, and preferably 15 to 25 bp. In general, the number of bases between the primers is suitably 300 to 2000 bp.

The reaction conditions for PCR are not particularly limited but may be, for example, a denaturation temperature of 90 to 95°C, an annealing temperature of 40 to 60°C, an elongation temperature of 60 to 75°C, and the number of cycle of 10 or more. The resulting reaction product may be separated, for example, by electrophoresis using agarose gel to determine the molecular weight of the amplified product. This method allows prediction and assessment of the capability of the yeast to produce hydrogen sulfide as determined by whether the molecular weight of the amplified product is a size that contains the DNA molecule of the specific part. In addition, by analyzing the nucleotide sequence of the amplified product, the capability may be predicted and/or assessed more precisely.

Moreover, in the present invention, a test yeast is cultured to measure an expression level of the O-acetylhomoserinesulfhydrylase gene having the nucleotide sequence of SEQ ID NO: 1 to assess the test yeast for its hydrogen sulfide-producing capability. In measuring an expression level of the O-acetylhomoserinesulfhydrylase gene, the test yeast is cultured and then mRNA or a protein resulting from the O-acetylhomoserinesulfhydrylase gene is quantified. The quantification of mRNA or protein may be carried out by employing a known technique. For example, mRNA may be quantified, by Northern hybridization or quantitative RT-PCR, while protein may be quantified, for example, by Western blotting (CURRENT PROTOCOLS IN MOLECULAR BIOLOGY, John Wiley & Sons 1994-2003).

Furthermore, test yeasts are cultured and expression levels of the O-acetylhomoserinesulfhydrylase gene having the nucleotide sequence of SEQ ID NO: 1 are measured to select a test yeast with the gene expression level according to the target capability of producing hydrogen sulfide, thereby selecting a yeast favorable for brewing desired alcoholic beverages. In addition, a reference yeast and a test yeast may be cultured so as to measure and compare the expression level of the gene in each of the yeasts, thereby selecting a favorable test yeast. More specifically, for example, a reference yeast and one or more test yeasts are cultured and an expression level of the O-acetylhomoserinesulfhydrylase gene having the nucleotide sequence of SEQ ID NO: 1 is measured in each yeast By selecting a test yeast with the gene expressed higher than that in the reference yeast, a yeast suitable for brewing alcoholic beverages can be selected.

Alternatively, test yeasts are cultured and a yeast with a lower hydrogen sulfide-producing capability or with a higher or lower O-acetylhomoserinesulfhydrylase activity is selected, thereby selecting a yeast suitable for brewing desired alcoholic beverages.

In these cases, the test yeasts or the reference yeast may be, for example, a yeast introduced with the vector of the invention, a yeast in which an expression of a polynucleotide (DNA) of the invention has been controlled, an artificially mutated yeast or a naturally mutated yeast. The production amount of hydrogen sulfide can be measured by, for example, any of the methods described in Brauwissenschaft. 31. 1 (1978), Applied Environm. Microbiol. 66:4421-4426 (2000), or J. Am. Soc. Brew. Chem. 53: 58-62 (1995). O-acetylhomoserinesulfhydrylase activity can be measured by, for example, a method described in Yeast. 9 (12): 1335-42, 1993. The mutation treatment may employ any methods including, for example, physical methods such as ultraviolet irradiation and radiation irradiation, and chemical methods associated with treatments with drugs such as EMS (ethylmethane sulphonate) and N-methyl-N-nitrosoguanidine (*see, e.g*., Yasuji Oshima Ed., BIOCHEMISTRY EXPERIMENTS vol 39, Yeast Molecular Genetic Experiments, pp. 67-75, JSSP).

In addition, examples of yeasts used as the reference yeast or the test yeasts include any yeasts that can be used for brewing, for example, brewery yeasts for beer, wine, sake and the like. More specifically, yeasts such as genus *Sacchammyces* may be used (*e.g., S. pastorianus, S. cerevisiae,* and *S*. *carlsbergensis*)*.* According to the present invention, a lager brewing yeast, for example, *Saccharomyces pastorianus* W34/70; *Saccharomyces carlsbergensis* NCYC453 or NCYC456; or *Saccharomyces cerevisiae* NBRC1951, NBRC1952, NBRC1953 or NBRC1954 may be used Further, wine yeasts such as wine yeasts #1, 3 and 4 from the Brewing Society of Japan; and sake-yeasts such as sake yeast #7 and 9 from the Brewing Society of Japan may also be used but not limited thereto. In the present invention, lager brewing yeasts such as *Saccharomyces pastorianus* may preferably be used. The reference yeast and the test yeasts may be selected from the above yeasts in any combination.

### EXAMPLES

Hereinafter, the present invention will be described in more detail with reference to working examples. The present invention, however, is not limited to the examples described below.

### Example 1: Cloning of O-acetylhomoserinesulfhydrylase (non-ScMET17) Gene

A specific novel O-acetylhomoserinesulfhydrylase gene (non-ScMET17) gene (SEQ ID NO: 1) from a lager brewing yeast were found, as a result of a search utilizing the comparison database described in Japanese Patent Application Laid-Open No. 2004-283169. Based on the acquired nucleotide sequence information, primers non-ScMET17_for (SEQ ID NO: 3) and non ScMET17_rv (SEQ ID NO: 4) were designed to amplify the full-length genes, respectively. PCR was carried out using chromosomal DNA of a genome sequencing strain, *Saccharomyces pastorianus* Weihenstephan 34/70 strain, as a template to obtain- DNA fragments (about 1.3 kb) including the full-length gene of non-ScMET17.

The thus-obtained non-ScMET17 gene fragment was inserted into pCR21-TOPO vector (Invitrogen) by TA cloning. The nucleotide sequences of non-ScMET17 gene were analyzed according to Sanger's method (F. Sanger, Science, 214: 1215, 1981) to confirm the nucleotide sequence.

### Example 2: Analysis of Expression of non-ScMET17 Gene during Beer Fermentation

A beer fermentation test was conducted using a lager brewing yeast, *Saccharomyces pastorianus* Weihenstephan 34/70 strain and then mRNA extracted from yeast cells during fermentation was analyzed by a DNA microarray.

| | |
|---|---|
| Wort extract concentration | 12.69% |
| Wort content | 70L |
| Wort dissolved oxygen concentration | 8.6 ppm |
| Fermentation temperature | 15°C |
| Yeast pitching rate | 12.8x10⁶ cells/mL |

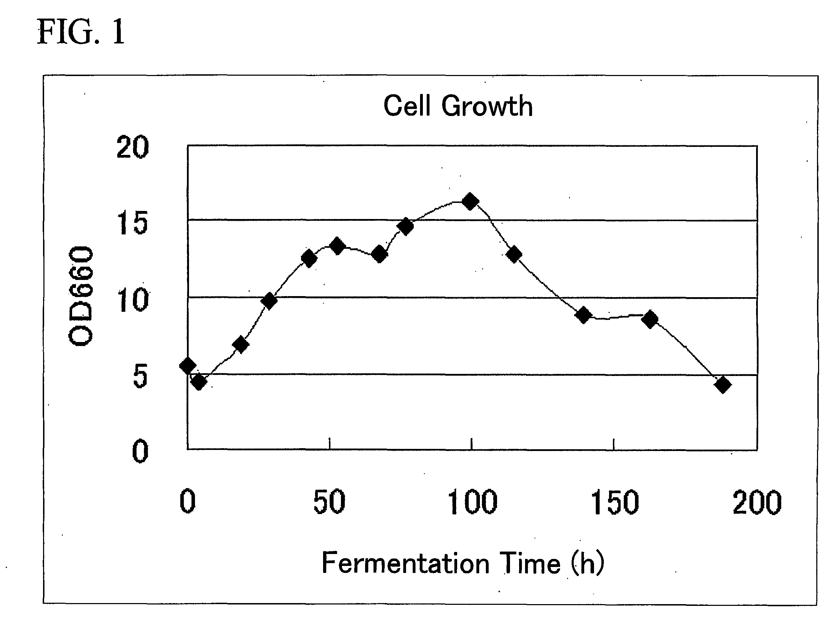

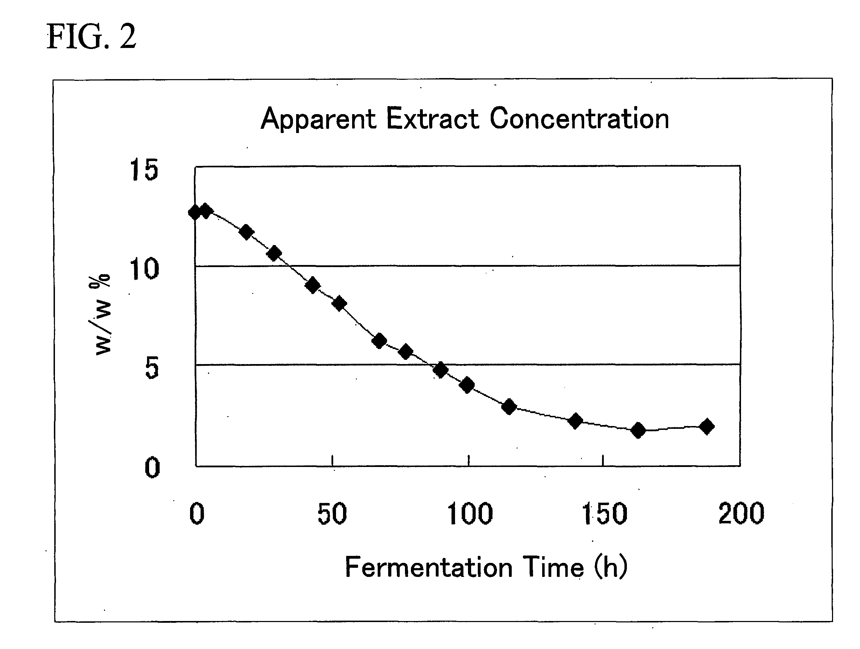

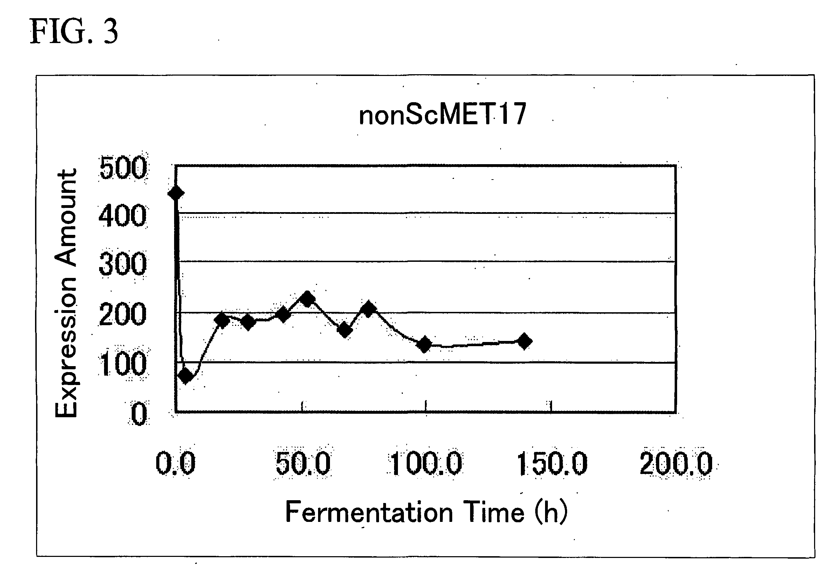

Sampling of fermentation liquor was performed with time, and variation with time of yeast growth amount (Fig. 1) and apparent extract concentration (Fig. 2) was observed. Simultaneously, sampling of yeast cells was performed, and the prepared mRNA was subjected to be biotin-labeled and was hybridized to a beer yeast DNA microarray. The signal was detected using GCOS; GeneChip Operating Software 1.0 (manufactured by Affymetrix Co.). Expression pattern of non-ScMET17 gene is shown in Figure 3. As a result, it was confirmed that non-ScMET17 gene was expressed in the general beer fermentation.

### Example 3: High Expression of non-ScMET17 Gene

The non-ScNMT17/pCR2.1-TOPO described in Example 1 was digested using the restriction enzymes SacI and NotI so as to prepare a DNA fragment containing the entire length of the protein-encoding region. This fragment was ligated to pYCGPYNot treated with the restriction enzymes SacI and NotI, thereby constructing the non-ScMET17 high expression vector non-ScNMT17/pYCGPYNot. pYCGPYNot is the YCp-type yeast expression vector. The inserted gene is highly expressed by the pyruvate kinase gene PYK1 promoter. The geneticin-resistant gene G418^{r} is included as the selection marker in the yeast, and the ampicillin-resistant gene Amp^{r} is included as the selection marker in *Escherichia coli.*

Using the high expression vector prepared by the above method, the strain *Saccharomyces pasteurianus* Weihenstephaner 34/70 was transformed by the method described in Japanese Patent Application Laid-open No. H7-303475. The transformant was selected in a YPD plate culture (1% yeast extract, 2% polypeptone, 2% glucose, 2% agar) containing 300 mg/L of geneticin.

### Example 4: Analysis of Amount of Hydrogen Sulfide Produced in Test Brewing of Beer

A fermentation test was carried out under the following conditions using the parent strain and the non-ScMET17 highly expressed strain obtained in Example 3.

| | |
|---|---|
| Wort extract concentration | 12% |
| Wort content | 1 L |
| Wort dissolved oxygen concentration | approx. 8 ppm |
| Fermentation temperature | 15°C (fixed) |
| Yeast pitching rate | 5 g wet yeast cells/L of wort |

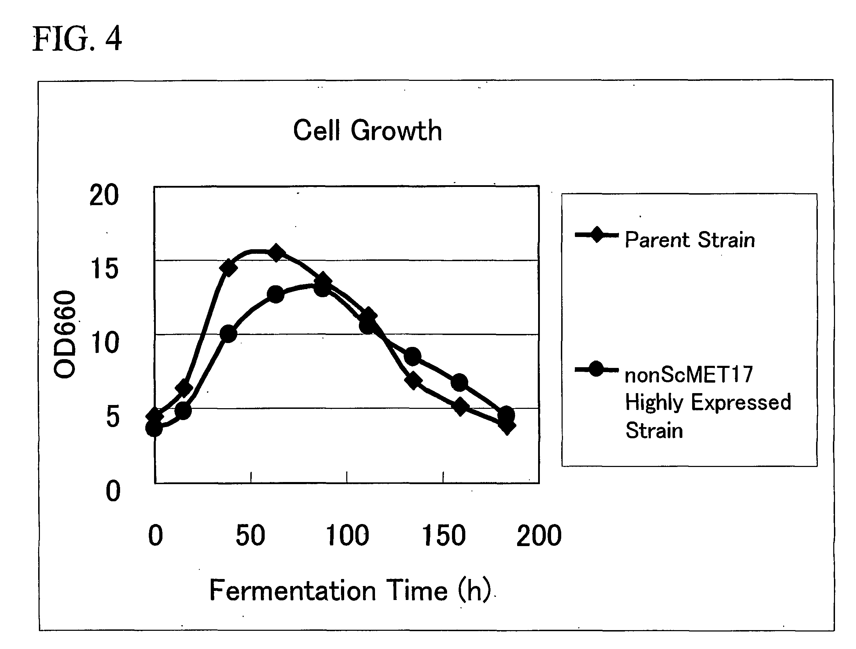

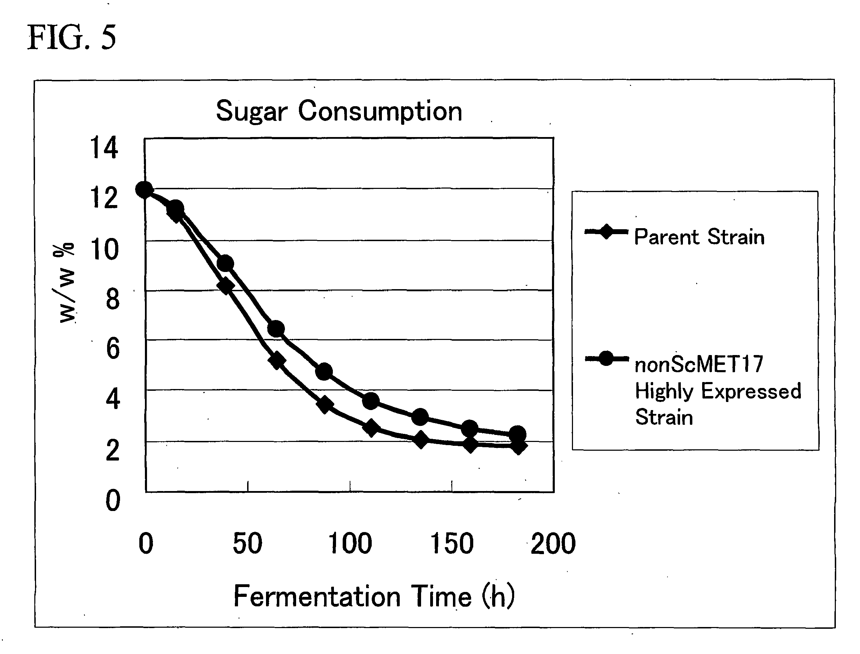

The fermentation broth was sampled over time, and the change over time in the yeast growth rate (OD660) (see FIG. 4) and the amount of extract consumed were determined (see FIG. 5). Quantitative determination of the hydrogen sulfide during fermentation was carried out based on the method of Takahashi et al. (Brauwissenschaft 31,1 (1978)). First, a sample containing a known concentration of hydrogen sulfide was measured and a standard curve for hydrogen sulfide was prepared from the peak surface area for the hydrogen sulfide detected. The amount of hydrogen sulfide was determined from the relationship between the standard curve and the surface area for the hydrogen sulfide detected in measurement of the fermentation broth under the same conditions as those used for analyzing the standard sample.

**Table 1. Amount of hydrogen sulfide in fermentation broth at completion of fermentation.**

| | Parent strain | non-ScMET17 highly expressed strain |
|---|---|---|
| H₂S (ppb) | 22.1 | -- |

| | | |
|---|---|---|
| Note: A dash (--) indicates that the result was below the limit of detection (a H₂S peak was not detected). | | |

From Table 1, the amount of hydrogen sulfide that had been produced on the completion of fermentation was 22.1 ppb for the parent strain, and was below the limit of detection for the non-ScMET17 highly expressed strain. It is clear from these results that the amount of hydrogen sulfide produced decreases significantly with high expression of the non-ScMET17 gene.

### Industrial Applicability

The inventive method of producing alcoholic beverages, by holding to a low level the concentration of hydrogen sulfide in beer fermentation and the finished product, can be used to produce alcoholic beverages having an excellent flavor.

This application claims benefit of Japanese Patent Application Nos. 2005-240351 filed August 22, 2005 and 2006-47564 filed February 23, 2006.

## Claims

1. A polynucleotide comprising the nucleotide sequence of SEQ ID NO: 1.

2. The polynucleotide of Claim 1 comprising a polynucleotide encoding a protein consisting of SEQ ID NO: 2.

3. The polynucleotide of Claim 1 or 2, wherein the polynucleotide is DNA.

4. A protein encoded by the polynucleotide of any one of Claims 1 to 3.

5. A vector comprising the polynucleotide of any one of Claims 1 to 3.

6. A yeast comprising the vector of Claim 5.

7. The yeast of Claim 6, wherein a hydrogen sulfide-producing ability is reduced by introducing the vector of Claim 5.

8. The yeast of Claim 7, wherein a hydrogen sulfide-producing ability is reduced by increasing an expression level of the protein of Claim 4.

9. A method for producing an alcoholic beverage comprising culturing the yeast of any one of Claims 6 to 8.

10. The method for producing an alcoholic beverage of Claim 9, wherein the brewed alcoholic beverage is a malt beverage.

11. The method for producing an alcoholic beverage of Claim 9, wherein the brewed alcoholic beverage is wine.

12. A method for assessing a test yeast for its hydrogen sulfide-producing capability, comprising a step of designing a primer or a probe based on the nucleotide sequence of SEQ ID NO: 1 and a step of using the primer or probe designed to assess the test yeast for its hydrogen sulfide-producing capability.

13. A method for assessing a test yeast for its hydrogen sulfide-producing capability, comprising: culturing a test yeast; and measuring an expression level of an O-acetylhomoserinesulfhydrylase gene having the nucleotide sequence of SEQ ID NO: 1.

14. A method for selecting a yeast, comprising: culturing test yeasts; quantifying the protein according to Claim 4 or measuring an expression level of an O-acetylhomoserinesulfhydrylase gene having the nucleotide sequence of SEQ ID NO: 1; and selecting a test yeast having said protein amount or said gene expression level according to a target capability of producing hydrogen sulfide.

15. The method for selecting a yeast according to Claim 14, comprising: culturing a reference yeast and test yeasts; measuring an expression level of an O-acetylhomoserinesulfhydrylase gene having the nucleotide sequence of SEQ ID NO: 1 in each yeast; and selecting a test yeast having the gene expressed higher than that in the reference yeast.

16. The method for selecting a yeast according to Claim 14, comprising: culturing a reference yeast and test yeasts; quantifying the protein according to Claim 4 in each yeast; and selecting a test yeast having said protein for a larger amount than that in the reference yeast.

17. A method for producing an alcoholic beverage comprising: conducting fermentation for producing an alcoholic beverage using the yeast according to any one of Claims 6 to 8; and adjusting the production amount of hydrogen sulfide.

## Patentansprüche

1. Polynukleotid umfassend die Nukleotidsequenz von SEQ ID NO: 1.

2. Polynukleotid nach Anspruch 1 umfassend ein Polynukleotid, welches ein Protein bestehend aus SEQ ID NO: 2 kodiert.

3. Polynukleotid nach Anspruch 1 oder 2, wobei das Polynukleotid DNA ist.

4. Protein, das durch das Polynukleotid nach einem der Ansprüche 1 bis 3 kodiert wird.

5. Vektor umfassend das Polynukleotid nach einem der Ansprüche 1 bis 3.

6. Hefe umfassend den Vektor nach Anspruch 5.

7. Hefe nach Anspruch 6, wobei die Fähigkeit, Schwefelwasserstoff zu erzeugen, durch das Einführen des Vektors nach Anspruch 5 verringert ist.

8. Hefe nach Anspruch 7, wobei die Fähigkeit, Schwefelwasserstoff zu erzeugen, durch Erhöhung des Expressionsspiegels des Proteins nach Anspruch 4 verringert ist.

9. Verfahren zur Herstellung eines alkoholischen Getränks umfassend das Züchten der Hefe nach einem der Ansprüche 6 bis 8.

10. Verfahren zur Herstellung eines alkoholischen Getränks nach Anspruch 9, wobei das gebraute alkoholische Getränk ein Malz-Getränk ist.

11. Verfahren zur Herstellung eines alkoholischen Getränks nach Anspruch 9, wobei das vergorene alkoholische Getränk Wein ist.

12. Verfahren zur Bewertung der Fähigkeit einer Test-Hefe, Schwefelwasserstoff zu erzeugen, umfassend einen Schritt, in dem ein Primer oder eine Sonde auf der Basis der Nukleotidsequenz von SEQ ID NO: 1 entworfen wird, und einen Schritt, in dem der entworfene Primer oder die entworfene Sonde für die Bewertung der Fähigkeit einer Test-Hefe, Schwefelwasserstoff zu erzeugen, verwendet wird.

13. Verfahren zur Bewertung der Fähigkeit einer Test-Hefe, Schwefelwasserstoff zu erzeugen, umfassend: Züchten einer Test-Hefe; und Messen eines Expressionsspiegels eines O-Acetylhomoserinsulfhydrylase-Gens, welches die Nukleotidsequenz von SEQ ID NO: 1 besitzt.

14. Verfahren zur Auswahl einer Hefe umfassend: das Züchten von Test-Hefen; das Quantifizieren des Proteins nach Anspruch 4 oder das Messen eines Expressionsspiegels eines O-Acetylhomoserinsulfhydrylase-Gens, welches die Nukleotidsequenz von SEQ ID NO: 1 besitzt; und das Auswählen einer Test-Hefe, welche die Proteinmenge oder den Genexpressionsspiegel gemäß der gewünschten Fähigkeit, Schwefelwasserstoff zu erzeugen, aufweist.

15. Verfahren zur Auswahl einer Hefe nach Anspruch 14, umfassend: das Züchten einer Referenz-Hefe und von Test-Hefen; das Messen des Expressionsspiegels eines O-Acetylhomoserinsulfhydrylase-Gens, welches die Nukleotidsequenz von SEQ ID NO: 1 besitzt, in jeder Hefe; und das Auswählen einer Test-Hefe, in der das Gen höher exprimiert ist als in der Referenz-Hefe.

16. Verfahren zur Auswahl einer Hefe nach Anspruch 14, umfassend: das Züchten einer Referenz-Hefe und von Test-Hefen; das Quantifizieren des Proteins nach Anspruch 4 in jeder Hefe; und das Auswählen einer Test-Hefe, in der das Protein in größerer Menge als in der Referenz-Hefe vorkommt.

17. Verfahren zur Herstellung eines alkoholischen Getränks umfassend: das Durchführen einer Fermentation zur Herstellung eines alkoholischen Getränks unter Verwendung der Hefe nach einem der Ansprüche 6 bis 8; und das Anpassen des Umfangs der Produktion von Schwefelwasserstoff.

## Revendications

1. Polynucléotide comprenant la séquence nucléotidique de SEQ ID NO : 1.

2. Polynucléotide selon la revendication 1 comprenant un polynucléotide codant une protéine constituée de SEQ ID NO :2.

3. Polynucléotide selon la revendication 1 ou 2, dans lequel le polynucléotide est un ADN.

4. Protéine codée par le polynucléotide selon l'une quelconque des revendications 1 à 3.

5. Vecteur comprenant le polynucléotide selon l'une quelconque des revendications 1 à 3.

6. Levure comprenant le vecteur selon la revendication 5.

7. Levure selon la revendication 6, dans laquelle une capacité de production de sulfure d'hydrogène est réduite en introduisant le vecteur selon la revendication 5.

8. Levure selon la revendication 7, dans laquelle une capacité de production de sulfure d'hydrogène est réduite en augmentant un niveau d'expression de la protéine selon la revendication 4.

9. Méthode de production d'une boisson alcoolisée comprenant la culture de la levure selon l'une quelconque des revendications 6 à 8.

10. Méthode de production d'une boisson alcoolisée selon la revendication 9, dans laquelle la boisson alcoolisée brassée est une boisson de malt.

11. Méthode de production d'une boisson alcoolisée selon la revendication 9, dans laquelle la boisson alcoolisée brassée est du vin.

12. Méthode d'évaluation d'une levure de test pour sa capacité à produire du sulfure d'hydrogène, comprenant une étape de conception d'une amorce ou d'une sonde basée sur la séquence nucléotidique de SEQ ID NO :1 et une étape d'utilisation de l'amorce ou de la sonde conçue pour évaluer la levure de test pour sa capacité à produire du sulfure d'hydrogène.

13. Méthode d'évaluation d'une levure de test pour sa capacité à produire du sulfure d'hydrogène, comprenant : la culture d'une levure de test ; et la mesure d'un niveau d'expression d'un gène de O-acétylhomosérinesulfhydrylase ayant la séquence nucléotidique de SEQ ID NO :1.

14. Méthode de sélection d'une levure, comprenant : la culture de levures de test ; la quantification de la protéine selon la revendication 4 ou la mesure d'un niveau d'expression d'un gène de O-acétylhomosérinesulfhydrylase ayant la séquence nucléotidique de SEQ ID NO :1 ; et la sélection d'une levure de test ayant une quantité de ladite protéine ou un niveau d'expression dudit gène conformes à une capacité à produire du sulfure d'hydrogène cible.

15. Méthode de sélection d'une levure selon la revendication 14, comprenant : la culture d'une levure de référence et de levures de test ; la mesure d'un niveau d'expression d'un gène de O-acétylhomosérinesulfhydrylase ayant la séquence nucléotidique de SEQ ID NO :1 dans chaque levure ; et la sélection d'une levure de test ayant le gène exprimé de façon plus importante que dans la levure de référence.

16. Méthode de sélection d'une levure selon la revendication 14, comprenant : la culture d'une levure de référence et de levures de test ; la quantification de la protéine selon la revendication 4 dans chaque levure ; et la sélection d'une levure de test ayant ladite protéine en une quantité plus importante que dans la levure de référence.

17. Méthode de production d'une boisson alcoolisée comprenant : la conduite d'une fermentation pour produire une boisson alcoolisée en utilisant la levure selon l'une quelconque des revendications 6 à 8 ; et l'ajustement de la quantité de production de sulfure d'hydrogène.


### SEQUENCE LISTING

<110> Suntory Limited
<120> O-acetylhomoserinesulfhydorelace gene and use thereof
<130> PCT06-0076
<150> JP2005-240351
   <151> 2005-08-22
<150> JP2006-47564
   <151> 2006-02-23
<160> 4
<170> PatentIn version 3.1
<210> 1
   <211> 1335
   <212> DNA
   <213> yeast
<400> 1
   atgccatctc atttcgatac tgttcaatta cacgctggtc aagaggaccc tagtgacaat      60
   gctcacagaa caagagctgt cccaatctac gccactagtt cttacgtctt tgaaaactct     120
   aagcatggtt ctcaattgtt tggcctagaa gtgccaggtt acgtttattc tcgtttccaa     180
   aatcctacca gtaacgtttt ggaggaaaga atcgctgctt tagaaggtgg tgctgctgct     240
   ttagccgttt cctctggtca ggctgcccaa actcttgcca ttcaaggttt ggctcacact     300
   ggtgacaaca ttgtctccac ttcttactta tatggtggta cttacaacca attcaaaatc     360
   tcattcaaaa gattcggtat cgaagccaga tttgtcgaag gtgacaatcc agaagacttc     420
   gaaaaggtct tcgatgaaag aaccaaggct gtttacttgg aaactattgg taatccaagt 480
   tataatgttc cagatttcga aaagattgtt gccattgctc acaaacatgg tattccagtt 540
   gttgttgata acacattcgg tgctggtggt ttcttctgtc aacctattaa gtgcggtgct 600
   gatattgtaa cacactctgc taccaagtgg attggtggtc acggtaccac catcggtggt 660
   attattgttg actctggtaa gttcccatgg aaggactacc cagaaaagtt cccacaattc 720
   tctcaaccgg ccgaaggcta ccacggtact atatacaacg aagcctacgg taacttggct 780
   tacattgttc atgttagaac tgaactgtta agagatttgg gtccattgat gaacccattt 840
   gcctctttcc tactactaca aggtgtcgaa acattatctt tgagagctga aagacacggt 900
   gaaaatgcct tgaagttggc caaatggttg gagcagtctc cttacgtatc ctgggtatcc 960
   taccctggtt tagcatctca ttctcatcat gaaaacgcta aaaagtatct atcaaacggt 1020
   ttcggtggtg tcttatcctt cggtgttaag gacttgccaa acgctgacaa ggaaactgat 1080
   ccattcaaac tttccggtgc ccaagttgtt gacggtttga agcttgcttc caacttggcc 1140
   aacgttggtg atgccaaaac tttagtcatt gctccatact ttaccaccca caagcaatta 1200
   aatgacaagg aaaagttggc ttctggtgtc accaaggact tgattcgtgt atctgttggt 1260
   atcgaattca ttgatgatat tattgcagac ttccaacaat ctcttgaaac tgttttcgct 1320
   ggtcaaaaac cataa 1335
<210> 2
   <211> 444
   <212> PRT
   <213> yeast
<400> 2
   Met Pro Ser His Phe Asp Thr Val Gln Leu His Ala Gly Gln Glu Asp
   1 5 10 15
   Pro Ser Asp Asn Ala His Arg Thr Arg Ala Val Pro Ile Tyr Ala Thr
   20 25 30
   Ser Ser Tyr Val Phe Glu Asn Ser Lys His Gly Ser Gln Leu Phe Gly
   35 40 45
   Leu Glu Val Pro Gly Tyr Val Tyr Ser Arg Phe Gln Asn Pro Thr Ser
   50 55 60
   Asn Val Leu Glu Glu Arg Ile Ala Ala Leu Glu Gly Gly Ala Ala Ala
   65 70 75 80
   Leu Ala Val Ser Ser Gly Gln Ala Ala Gln Thr Leu Ala Ile Gln Gly
   85 90 95
   Leu Ala His Thr Gly Asp Asn Ile Val Ser Thr Ser Tyr Leu Tyr Gly
   100 105 110
   Gly Thr Tyr Asn Gln Phe Lys Ile Ser Phe Lys Arg Phe Gly Ile Glu
   115 120 125
   Ala Arg Phe Val Glu Gly Asp Asn Pro Glu Asp Phe Glu Lys Val Phe
   130 135 140
   Asp Glu Arg Thr Lys Ala Val Tyr Leu Glu Thr Ile Gly Asn Pro Ser
   145 150 155 160
   Tyr Asn Val Pro Asp Phe Glu Lys Ile Val Ala Ile Ala His Lys His
   165 170 175
   Gly Ile Pro Val Val Val Asp Asn Thr Phe Gly Ala Gly Gly Phe Phe
   180 185 190
   Cys Gln Pro Ile Lys Cys Gly Ala Asp Ile Val Thr His Ser Ala Thr
   195 200 205
   Lys Trp Ile Gly Gly His Gly Thr Thr Ile Gly Gly Ile Ile Val Asp
   210 215 220
   Ser Gly Lys Phe Pro Trp Lys Asp Tyr Pro Glu Lys Phe Pro Gln Phe
   225 230 235 240
   Ser Gln Pro Ala Glu Gly Tyr His Gly Thr Ile Tyr Asn Glu Ala Tyr
   245 250 255
   Gly Asn Leu Ala Tyr Ile Val His Val Arg Thr Glu Leu Leu Arg Asp
   260 265 270
   Leu Gly Pro Leu Met Asn Pro Phe Ala Ser Phe Leu Leu Leu Gln Gly
   275 280 285
   Val Glu Thr Leu Ser Leu Arg Ala Glu Arg His Gly Glu Asn Ala Leu
   290 295 300
   Lys Leu Ala Lys Trp Leu Glu Gln Ser Pro Tyr Val Ser Trp Val Ser
   305 310 315 320
   Tyr Pro Gly Leu Ala Ser His Ser His His Glu Asn Ala Lys Lys Tyr
   325 330 335
   Leu Ser Asn Gly Phe Gly Gly Val Leu Ser Phe Gly Val Lys Asp Leu
   340 345 350
   Pro Asn Ala Asp Lys Glu Thr Asp Pro Phe Lys Leu Ser Gly Ala Gln
   355 360 365
   Val Val Asp Gly Leu Lys Leu Ala Ser Asn Leu Ala Asn Val Gly Asp
   370 375 380
   Ala Lys Thr Leu Val Ile Ala Pro Tyr Phe Thr Thr His Lys Gln Leu
   385 390 395 400
   Asn Asp Lys Glu Lys Leu Ala Ser Gly Val Thr Lys Asp Leu Ile Arg
   405 410 415
   Val Ser Val Gly Ile Glu Phe Ile Asp Asp Ile Ile Ala Asp Phe Gln
   420 425 430
   Gln Ser Leu Glu Thr Val Phe Ala Gly Gln Lys Pro
   435 440
<210> 3
   <211> 40
   <212> DNA
   <213> primer
<400> 3
   gagctcatag cggccatgcc atctcatttc gatactgttc 40
<210> 4
   <211> 42
   <212> DNA
   <213> primer
<400> 4
   ggatcctatg cggccgcaaa aaaggatatt catttcaata ac 42